# EUROPEAN PATENT APPLICATION

(11) **EP 4 509 593 A1**
(43) Date of publication of application: **19.02.2025**
(21) Application number: 23788634.6
(22) Date of filing: 14.04.2023
(51) Int. Cl.: C12N 1/04, C12N 7/00, A23K 10/16, A23L 29/00, A01N 63/40

(54) **NOVEL EXCIPIENT COMPOSITION FOR MAINTAINING LONG-TERM DISTRIBUTION STORAGE STABILITY OF BACTERIOPHAGES**

(30) Priority: 15.04.2022 KR 20220047176
(71) Applicant: CJ CheilJedang Corporation, Seoul 04560 (KR)
(72) Inventor: JEON, Jongsoo, Seoul 04560 (KR)
(74) Representative: Reddie & Grose LLP
(86) International application number: PCT/KR2023/005055
(87) International publication number: WO 2023/200285

(57) **Abstract**

The present invention relates to a novel composition for improving bacteriophage stability and maintaining long-term distribution storage stability of bacteriophages, the composition comprising sodium sulfate anhydrous so as to have excellent storage stability of bacteriophages.

## Description

### [TECHNICAL FIELD]

### Cross-reference to related application(s)

The present application claims the benefit of the priority based on Korean Patent Application No. 10-2022-0047176 filed on April 15, 2022, and the entire contents of the document of the corresponding Korean patent application are incorporated as a part of the present description.

Related are a novel excipient composition to maintain a long-term storage stability of bacteriophage and uses thereof.

### [BACKGROUND ART]

Foodborne disease caused by foodborne bacteria has been recognized as one of serious public health problems that are problems occurring worldwide. Therefore, in the food industry, it is important not only to provide delicious and nutritious food to consumers, but also to supply safe food in which foodborne bacteria which can cause disease do not exist.

Bacteriophage is one type of viruses that use bacteria as a host cell, and does not show human body toxicity because it specifically binds only to bacteria. Due to this characteristic, the bacteriophage has been widely used in clinical treatment of infections caused by bacteria, and in particular, has been used as a feed additive for preventing or inhibiting livestock disease (Korean Patent Publication No. 10-2021-0145038).

On the other hand, excipients used conventionally have a problem of deterioration of the productivity and product quality due to decrease in bacteriophage titer, during long-term storage according to production and distribution of bacteriophage products for feed addition. Therefore, when bacteriophage products for feed addition are distributed for a long period of time, research on an excipient for quality maintenance is necessary.

### [Prior art]

(Patent document 1) KR 10-2021-0145038 A

### [DISCLOSURE]

### [TECHNICAL PROBLEM]

One embodiment of the present application provides a composition for improving stability of bacteriophage comprising anhydrous sodium sulfate. The composition may be for maintaining and/or increasing stability of bacteriophage (for example, storage stability, etc.) for a long period of time.

Another embodiment provides a composition comprising a bacteriophage and anhydrous sodium sulfate. The composition may be a feed composition, food composition, pharmaceutical composition, or antibacterial composition.

Other embodiment provides a method for improving stability of bacteriophage, comprising mixing a bacteriophage and a composition for improving stability of bacteriophage comprising anhydrous sodium sulfate.

Other embodiment provides a use of anhydrous sodium sulfate for use in improvement of stability of bacteriophage, or manufacture of a composition for improving stability of bacteriophage.

Other embodiment provides a use of a bacteriophage and anhydrous sodium sulfate for use in manufacture of a feed composition, food composition, pharmaceutical composition, or antibacterial composition.

Other embodiment provides a use of a bacteriophage and a composition for improving stability of bacteriophage for use in manufacture of a feed composition, food composition, pharmaceutical composition, or antibacterial composition.

### [TECHNICAL SOLUTION]

One aspect, provides a composition for improving stability of bacteriophage comprising anhydrous sodium sulfate.

Anhydrous sodium sulfate (Sodium sulfate anhydrous, Na₂SO₄, 142.04 g/mol, CAS Number: 7757-82-6) is a sulphate of sodium, and a colorless crystal. The anhydrous sodium sulfate binds to 10 external water molecules and turns into sodium sulfate (Na₂SO₄·10H₂O; 322.20 g/mol), so it can play a role of moisture absorption in a moist environment.

The composition for improving stability of bacteriophage may further comprise an excipient component, or may be used as an excipient.

The excipient may mean a substance added to make feed, food or medicine easy to eat or to make a certain shape. As types of the excipient, there are a binder which facilitates molding by giving binding force to powder raw materials, a disintegrant that promotes disintegration, a lubricant added to ensure smooth compression operation and good flowability of granular products, an adsorbent that absorbs liquid raw materials and make them into tablets (pills), and the like. At least one selected from the group consisting of hydroxypropylmethyl cellulose (HPMC), hydroxypropyl cellulose (HPC), lactose (hydrous, anhydrous, monohydrate, spray-dried form, etc.), microcrystalline cellulose, titanium dioxide, silicon dioxide (colloidal, colloidal silicon dioxide, etc.), magnesium stearate, croscarmellose sodium, alpha starch (starch pregelatinized), sodium starch glycolate, crosspovidone, talc, wheat flour (grain powder, wheat powder), calcium carbonate, silica, and the like may be illustrated as a mainly used excipient component, but not limited thereto. The composition for improving stability of bacteriophage provided in the present application may further comprise at least one of the excipient components in addition to anhydrous sodium sulfate.

The ratio of anhydrous sodium sulfate comprised in the composition for improving stability of bacteriophage may be 1 to 50%(w/w), 1 to 45%(w/w), 1 to 40%(w/w), 1 to 35%(w/w), 1 to 30%(w/w), 5 to 50%(w/w), 5 to 45%(w/w), 5 to 40%(w/w), 5 to 35%(w/w), 5 to 30%(w/w), 10 to 50%(w/w), 10 to 45%(w/w), 10 to 40%(w/w), 10 to 35%(w/w), 10 to 30%(w/w), 15 to 50%(w/w), 15 to 45%(w/w), 15 to 40%(w/w), 15 to 35%(w/w), 15 to 30%(w/w), 20 to 50%(w/w), 20 to 45%(w/w), 20 to 40%(w/w), 20 to 35%(w/w), 20 to 30%(w/w), 25 to 50%(w/w), 25 to 45%(w/w), 25 to 40%(w/w), 25 to 35%(w/w), 25 to 30%(w/w), 30 to 50%(w/w), 30 to 45%(w/w), 30 to 40%(w/w), or 30 to 35%(w/w), or the like, based on the weight, but not limited thereto.

In the present description, the term "improving stability of bacteriophage" may maintain, improve or enhance storage stability of bacteriophage for a long period of time. In other words, the effect of improving stability of bacteriophage may mean an effect of maintaining titer of bacteriophage, or lowering a titer decrease.

Bacteriophage is a collective term for viruses that use bacteria and archaebacteria as a host, and may specifically infect bacteria. Accordingly, using an antibiotic effect of the bacteriophage, it is used for products such as feed (including feed additives), food, medicine, disinfectants and the like, but there is a problem of productivity and quality deterioration of products due to a decrease in the titer of the bacteriophage during storage according to production or distribution of the products. In other words, the effect of improving stability of bacteriophage may mean maintaining the titer of the bacteriophage during storage of products comprising such bacteriophage, or lowering a decrease in the titer of the bacteriophage.

In one embodiment, when the composition for improving stability of bacteriophage is applied into a bacteriophage (for example, mixing, etc.), when the bacteriophage is stored under a temperature condition of 45 to 60 °C, 45 to 58 °C, 45 to 55 °C, 45 to 52 °C, 45 to 50 °C, 45 to 48 °C, 48 to 60 °C, 48 to 58 °C, 48 to 55 °C, 48 to 52 °C, 48 to 50 °C, 50 to 60 °C, 50 to 58 °C, 50 to 55 °C, or 50 to 52 °C; and/or
under a relative humidity condition of 80 to 100%, 80 to 99%, 80 to 98%, 80 to 97%, 80 to 96%, 80 to 95%, 80 to 92%, 80 to 90%, 80 to 88%, 80 to 85%, 85 to 100%, 85 to 99%, 85 to 98%, 85 to 97%, 85 to 96%, 85 to 95%, 85 to 92%, 85 to 90%, 85 to 88%, 90 to 100%, 90 to 99%, 90 to 98%, 90 to 97%, 90 to 96%, 90 to 95%, 90 to 92%, 95 to 100%, 95 to 99%, 95 to 98%, 95 to 97%, or 95 to 96%,
compared to the titer of the bacteriophage measured on Day 0, the titer of the bacteriophage measured on Day 1 to Day 7, Day 1 to Day 14, Day 7 to Day 14, after Day 1, after Day 2, after Day 3, after Day 4, after Day 5, after Day 6, after Day 7, after Day 8, after Day 9, after Day 10, after Day 11, after Day 12, after Day 13, or after Day 14 may be reduced by 2 log(pfu/g) or less, 1.9 log(pfu/g) or less, 1.8 log(pfu/g) or less, 1.7 log(pfu/g) or less, 1.6 log(pfu/g) or less, 1.5 log(pfu/g) or less, or 1.4 log(pfu/g) or less, for example, 1.4 log(pfu/g), or
may be reduced by 20% or less, 19% or less, 18% or less, 17% or less, 16% or less, or 15% or less.

In another embodiment, when the composition for improving stability of bacteriophage is applied into a bacteriophage (for example, mixing, etc.), compared to a control group (for example, a case of not applying anhydrous sodium sulfate to the bacteriophage),
when the bacteriophage is stored under a temperature condition of 45 to 60 °C, 45 to 58 °C, 45 to 55 °C, 45 to 52 °C, 45 to 50 °C, 45 to 48 °C, 48 to 60 °C, 48 to 58 °C, 48 to 55 °C, 48 to 52 °C, 48 to 50 °C, 50 to 60 °C, 50 to 58 °C, 50 to 55 °C, or 50 to 52 °C; and/or
under a relative humidity condition of 80 to 100%, 80 to 99%, 80 to 98%, 80 to 97%, 80 to 96%, 80 to 95%, 80 to 92%, 80 to 90%, 80 to 88%, 80 to 85%, 85 to 100%, 85 to 99%, 85 to 98%, 85 to 97%, 85 to 96%, 85 to 95%, 85 to 92%, 85 to 90%, 85 to 88%, 90 to 100%, 90 to 99%, 90 to 98%, 90 to 97%, 90 to 96%, 90 to 95%, 90 to 92%, 95 to 100%, 95 to 99%, 95 to 98%, 95 to 97%, or 95 to 96%,
compared to the titer of the bacteriophage measured on Day 0, the titer decrement (log(pfu/g)) of the bacteriophage measured on Day 1 to Day 7, Day 1 to Day 14, Day 7 to Day 14, after Day 1, after Day 2, after Day 3, after Day 4, after Day 5, after Day 6, after Day 7, after Day 8, after Day 9, after Day 10, after Day 11, after Day 12, after Day 13, or after Day 14 may be lower as 0.5 to 3, 0.5 to 2.5, 0.5 to 2, 0.5 to 1.5, 0.6 to 3, 0.6 to 2.5, 0.6 to 2, 0.6 to 1.5, 0.7 to 3, 0.7 to 2.5, 0.7 to 2, 0.7 to 1.5, 0.8 to 3, 0.8 to 2.5, 0.8 to 2, 0.8 to 1.5, 0.9 to 3, 0.9 to 2.5, 0.9 to 2, 0.9 to 1.5, 1 to 3, 1 to 2.5, 1 to 2, 1 to 1.5, 0.5 or more, 0.6 or more, 0.7 or more, 0.8 or more, 0.9 or more, 1.0 or more, or 1.1 or more, for example, 1.1, or,
may be lower as 5 to 20%p, 5 to 15%p, 6 to 20%p, 6 to 15%p, 7 to 20%p, 7 to 15%p, 8 to 20%p, 8 to 15%p, 9 to 20%p, 9 to 15%p, 10 to 20%p, 10 to 15%p, 5%p or more, 6%p or more, 7%p or more, 8%p or more, 9%p or more, 10%p or more, or 11%p or more, for example, 11%p.

In other embodiment, when the composition for improving stability of bacteriophage is applied into a bacteriophage (for example, mixing, etc.), compared to a control group (for example, a case of not applying anhydrous sodium sulfate to the bacteriophage),
when the bacteriophage is stored under a temperature condition of 45 to 60 °C, 45 to 58 °C, 45 to 55 °C, 45 to 52 °C, 45 to 50 °C, 45 to 48 °C, 48 to 60 °C, 48 to 58 °C, 48 to 55 °C, 48 to 52 °C, 48 to 50 °C, 50 to 60 °C, 50 to 58 °C, 50 to 55 °C, or 50 to 52 °C; and/or
under a relative humidity condition of 80 to 100%, 80 to 99%, 80 to 98%, 80 to 97%, 80 to 96%, 80 to 95%, 80 to 92%, 80 to 90%, 80 to 88%, 80 to 85%, 85 to 100%, 85 to 99%, 85 to 98%, 85 to 97%, 85 to 96%, 85 to 95%, 85 to 92%, 85 to 90%, 85 to 88%, 90 to 100%, 90 to 99%, 90 to 98%, 90 to 97%, 90 to 96%, 90 to 95%, 90 to 92%, 95 to 100%, 95 to 99%, 95 to 98%, 95 to 97%, or 95 to 96% for 1 day to 7 days, 1 day to 14 days, 7 days to 14 days, 1 day, 2 days, 3 days, 4 days, 5 days, 6 days, 7 days, 8 days, 9 days, 10 days, 11 days, 12 days, 13 days, or 14 days,
the titer of the bacteriophage may be higher than 2 to 40%, 2 to 35%, 2 to 30%, 2 to 25%, 2 to 20%, 2 to 15%, 2 to 10%, 5 to 40%, 5 to 35%, 5 to 30%, 5 to 25%, 5 to 20%, 5 to 15%, 5 to 10%, 10 to 40%, 10 to 35%, 10 to 30%, 10 to 25%, 10 to 20%, 10 to 15%, 2% or more, 3% or more, 4% or more, 5% or more, 6% or more, 7% or more, 8% or more, 9% or more, 10% or more, 11% or more, 12% or more, 13% or more, 14% or more, or 15% or more.

When the composition for improving stability of bacteriophage is applied to a bacteriophage, the titer decrement of the bacteriophage is low even during long-term storage of the bacteriophage under an extreme environment such as high temperature and humidity condition, and the like, so there is an advantage in that the bacteriophage can be stably stored.

The composition for improving stability of bacteriophage may have an excellent effect of improving stability of bacteriophage, compared to a composition not comprising anhydrous sodium sulfate, by comprising anhydrous sodium sulfate.

The composition for improving stability of bacteriophage, may be used in manufacture of a feed composition, food composition, pharmaceutical composition, or antibacterial composition, or the like.

Another aspect, provides a composition, for example, a feed composition, food composition, pharmaceutical composition, or antibacterial composition, or the like, comprising a bacteriophage and anhydrous sodium sulfate. Otherwise, it provides a composition, for example, a feed composition, food composition, pharmaceutical composition, or antibacterial composition, or the like, comprising a bacteriophage and the composition for improving stability of bacteriophage.

As the bacteriophage, the kind and number of the bacteriophage are not particularly limited, and anything may be used, and one or more (for example, a combination of 2 kinds, 3 kinds) bacteriophage of bacteriophage belonging to the family of Myoviridae, Siphoviridae and Podoviridae may be selected and used.

The bacteriophage may be a bacteriophage which specifically infects bacteria belonging to the genus of *Escherichia* (for example, *E. coli, E. albertii, E. fergusonii, E. hermannii, E. vulneris,* etc.), the genus of *Salmonella* (for example, *S. bongori S. enterica,* etc.), or the genus of *Clostridium* (for example, *C. botulinum, C. tetani, C. perfringens, C. hisolyticum, C. sordellii, C. butyricum,* etc.), but not limited thereto.

The initial content (or, titer) of the bacteriophage comprised in the composition (for example, feed composition, food composition, pharmaceutical composition, or antibacterial composition, etc.) is not particularly limited, but it may be generally used in a range of 1×10¹ to 1×10¹⁵ pfu/g, and otherwise, the amount of the bacteriophage and anhydrous sodium sulfate (or composition for improving stability of bacteriophage) comprised in the composition, may be appropriately determined by those skilled in the art in consideration of a purpose of use of the finally prepared composition (for example, feed composition, food composition, pharmaceutical composition, or antibacterial composition, etc.).

In the composition (for example, feed composition, food composition, pharmaceutical composition, or antibacterial composition, etc.), the ratio of the composition for improving stability of bacteriophage in the bacteriophage and composition for improving stability of bacteriophage, may be 90 to 99.9%(w/w), 90 to 99.5%(w/w), 90 to 99%(w/w), 90 to 98%(w/w), 90 to 97%(w/w), 90 to 96%(w/w), 90 to 95%(w/w), 91 to 99.9%(w/w), 91 to 99.5%(w/w), 91 to 99%(w/w), 91 to 98%(w/w), 91 to 97%(w/w), 91 to 96%(w/w), 91 to 95%(w/w), 92 to 99.9%(w/w), 92 to 99.5%(w/w), 92 to 99%(w/w), 92 to 98%(w/w), 92 to 97%(w/w), 92 to 96%(w/w), 92 to 95%(w/w), 93 to 99.9%(w/w), 93 to 99.5%(w/w), 93 to 99%(w/w), 93 to 98%(w/w), 93 to 97%(w/w), 93 to 96%(w/w), 93 to 95%(w/w), 94 to 99.9%(w/w), 94 to 99.5%(w/w), 94 to 99%(w/w), 94 to 98%(w/w), 94 to 97%(w/w), 94 to 96%(w/w), 94 to 95%(w/w), 95 to 99.9%(w/w), 95 to 99.5%(w/w), 95 to 99%(w/w), 95 to 98%(w/w), 95 to 97%(w/w), or 95 to 96%(w/w), based on weight, but not limited thereto.

In the composition (for example, feed composition, food composition, pharmaceutical composition, or antibacterial composition, etc.), the mixing ratio of the bacteriophage and composition for improving stability of bacteriophage, may be 1: 8 to 1: 999, 1: 8 to 5: 995, 1: 8 to 1: 99, 1: 8 to 2: 98, 1: 8 to 3: 97, 1: 88 to 4: 96, 1: 8 to 5: 95, 1: 9 to 1: 999, 1: 9 to 5: 995, 1: 9 to 1: 99, 1: 9 to 2: 98, 1: 9 to 3: 97, 1: 9 to 4: 96, 1: 9 to 5: 95, or the like, based on weight, but not limited thereto.

In the composition, for example, a feed composition, food composition, pharmaceutical composition, or antibacterial composition, or the like, the ratio of anhydrous sodium sulfate in the bacteriophage and composition for improving stability of bacteriophage, may be 4 to 45%(w/w), 4 to 40%(w/w), 4 to 30%(w/w), 4 to 20%(w/w), 4 to 18%(w/w), 4 to 15%(w/w), 4 to 10%(w/w), 4 to 5%(w/w), 4 to 4.5%(w/w), 4.5 to 45%(w/w), 4.5 to 40%(w/w), 4.5 to 30%(w/w), 4.5 to 20%(w/w), 4.5 to 18%(w/w), 4.5 to 15%(w/w), 4.5 to 10%(w/w), 4.5 to 5%(w/w), 15 to 45%(w/w), 15 to 40%(w/w), 15 to 30%(w/w), 15 to 20%(w/w), 15 to 18%(w/w), 18 to 45%(w/w), 18 to 40%(w/w), 18 to 30%(w/w), 18 to 20%(w/w), 20 to 45%(w/w), 20 to 40%(w/w), 20 to 30%(w/w), 30 to 45%(w/w), 30 to 40%(w/w), or 40 to 45%(w/w), or the like, based on weight, but not limited thereto.

In one embodiment, when a composition, for example, feed composition, food composition, pharmaceutical composition or antibacterial composition or the like comprising the bacteriophage and anhydrous sodium sulfate or the composition for improving stability of bacteriophage,
is stored under a temperature condition of 45 to 60 °C, 45 to 58 °C, 45 to 55 °C, 45 to 52 °C, 45 to 50 °C, 45 to 48 °C, 48 to 60 °C, 48 to 58 °C, 48 to 55 °C, 48 to 52 °C, 48 to 50 °C, 50 to 60 °C, 50 to 58 °C, 50 to 55 °C, or 50 to 52 °C; and/or
under a relative humidity condition of 80 to 100%, 80 to 99%, 80 to 98%, 80 to 97%, 80 to 96%, 80 to 95%, 80 to 92%, 80 to 90%, 80 to 88%, 80 to 85%, 85 to 100%, 85 to 99%, 85 to 98%, 85 to 97%, 85 to 96%, 85 to 95%, 85 to 92%, 85 to 90%, 85 to 88%, 90 to 100%, 90 to 99%, 90 to 98%, 90 to 97%, 90 to 96%, 90 to 95%, 90 to 92%, 95 to 100%, 95 to 99%, 95 to 98%, 95 to 97%, or 95 to 96%,
compared to the titer of the bacteriophage measured on Day 0, the titer of the bacteriophage measured on Day 1 to Day 7, Day 1 to Day 14, Day 7 to Day 14, after Day 1, after Day 2, after Day 3, after Day 4, after Day 5, after Day 6, after Day 7, after Day 8, after Day 9, after Day 10, after Day 11, after Day 12, after Day 13, or after Day 14 may be reduced by 2 log(pfu/g) or less, 1.9 log(pfu/g) or less, 1.8 log(pfu/g) or less, 1.7 log(pfu/g) or less, 1.6 log(pfu/g) or less, 1.5 log(pfu/g) or less, or 1.4 log(pfu/g) or less, for example, 1.4 log(pfu/g), or,
may be reduced by 20% or less, 19% or less, 18% or less, 17% or less, 16% or less, or 15% or less.

In one embodiment, when a composition, for example, feed composition, food composition, pharmaceutical composition or antibacterial composition or the like comprising the bacteriophage and anhydrous sodium sulfate or the composition for improving stability of bacteriophage,
is stored under a temperature condition of 45 to 60 °C, 45 to 58 °C, 45 to 55 °C, 45 to 52 °C, 45 to 50 °C, 45 to 48 °C, 48 to 60 °C, 48 to 58 °C, 48 to 55 °C, 48 to 52 °C, 48 to 50 °C, 50 to 60 °C, 50 to 58 °C, 50 to 55 °C, or 50 to 52 °C; and/or
under a relative humidity condition of 80 to 100%, 80 to 99%, 80 to 98%, 80 to 97%, 80 to 96%, 80 to 95%, 80 to 92%, 80 to 90%, 80 to 88%, 80 to 85%, 85 to 100%, 85 to 99%, 85 to 98%, 85 to 97%, 85 to 96%, 85 to 95%, 85 to 92%, 85 to 90%, 85 to 88%, 90 to 100%, 90 to 99%, 90 to 98%, 90 to 97%, 90 to 96%, 90 to 95%, 90 to 92%, 95 to 100%, 95 to 99%, 95 to 98%, 95 to 97%, or 95 to 96%,
compared to the titer of the bacteriophage measured on Day 0, the titer decrement of the bacteriophage measured on Day 1 to Day 7, Day 1 to Day 14, Day 7 to Day 14, after Day 1, after Day 2, after Day 3, after Day 4, after Day 5, after Day 6, after Day 7, after Day 8, after Day 9, after Day 10, after Day 11, after Day 12, after Day 13, or after Day 14 may be lower as 0.5 to 3, 0.5 to 2.5, 0.5 to 2, 0.5 to 1.5, 0.6 to 3, 0.6 to 2.5, 0.6 to 2, 0.6 to 1.5, 0.7 to 3, 0.7 to 2.5, 0.7 to 2, 0.7 to 1.5, 0.8 to 3, 0.8 to 2.5, 0.8 to 2, 0.8 to 1.5, 0.9 to 3, 0.9 to 2.5, 0.9 to 2, 0.9 to 1.5, 1 to 3, 1 to 2.5, 1 to 2, 1 to 1.5, 0.5 or more, 0.6 or more, 0.7 or more, 0.8 or more, 0.9 or more, 1.0 or more, or 1.1 or more, for example, 1.1, or
may be lower as 5 to 20%p, 5 to 15%p, 6 to 20%p, 6 to 15%p, 7 to 20%p, 7 to 15%p, 8 to 20%p, 8 to 15%p, 9 to 20%p, 9 to 15%p, 10 to 20%p, 10 to 15%p, 5%p or more, 6%p or more, 7%p or more, 8%p or more, 9%p or more, 10%p or more, or 11%p or more, for example, 11%p.

In one embodiment, when a composition, for example, feed composition, food composition, pharmaceutical composition or antibacterial composition or the like comprising the bacteriophage and anhydrous sodium sulfate or the composition for improving stability of bacteriophage,
is stored under a temperature condition of 45 to 60 °C, 45 to 58 °C, 45 to 55 °C, 45 to 52 °C, 45 to 50 °C, 45 to 48 °C, 48 to 60 °C, 48 to 58 °C, 48 to 55 °C, 48 to 52 °C, 48 to 50 °C, 50 to 60 °C, 50 to 58 °C, 50 to 55 °C, or 50 to 52 °C; and/or
under a relative humidity condition of 80 to 100%, 80 to 99%, 80 to 98%, 80 to 97%, 80 to 96%, 80 to 95%, 80 to 92%, 80 to 90%, 80 to 88%, 80 to 85%, 85 to 100%, 85 to 99%, 85 to 98%, 85 to 97%, 85 to 96%, 85 to 95%, 85 to 92%, 85 to 90%, 85 to 88%, 90 to 100%, 90 to 99%, 90 to 98%, 90 to 97%, 90 to 96%, 90 to 95%, 90 to 92%, 95 to 100%, 95 to 99%, 95 to 98%, 95 to 97%, or 95 to 96% for 1 day to 7 days, 1 day to 14 days, 7 days to 14 days, 1 day, 2 days, 3 days, 4 days, 5 days, 6 days, 7 days, 8 days, 9 days, 10 days, 11 days, 12 days, 13 days, or 14 days,
compared to a composition comprising no anhydrous sodium sulfate,
the titer of the bacteriophage may be higher than 2 to 40%, 2 to 35%, 2 to 30%, 2 to 25%, 2 to 20%, 2 to 15%, 2 to 10%, 5 to 40%, 5 to 35%, 5 to 30%, 5 to 25%, 5 to 20%, 5 to 15%, 5 to 10%, 10 to 40%, 10 to 35%, 10 to 30%, 10 to 25%, 10 to 20%, 10 to 15%, 2% or more, 3% or more, 4% or more, 5% or more, 6% or more, 7% or more, 8% or more, 9% or more, 10% or more, 11% or more, 12% or more, 13% or more, 14% or more, or 15% or more.

When the composition (for example, feed composition, food composition, pharmaceutical composition or antibacterial composition, or the like) comprising the bacteriophage and anhydrous sodium sulfate or the composition for improving stability of bacteriophage has a low titer decrement of the bacteriophage even during long-term storage of the bacteriophage under an extreme environment such as high temperature and humidity condition, so there is an advantage in that the bacteriophage can be stably stored.

Accordingly, the composition comprising a bacteriophage and anhydrous sodium sulfate (or, a composition for improving stability of bacteriophage) may be prepared in various forms to suit a specific purpose in home, industrial, medical and environmental fields, and in one embodiment, it may be prepared in a form of a feed composition (includes a feed additive composition), food composition, pharmaceutical composition or antibacterial composition or the like, but not limited thereto.

Other aspect, provides a feed composition comprising a bacteriophage and anhydrous sodium sulfate (or, a composition for improving stability of bacteriophage).

In the present application, "feed" may mean any natural or artificial diet, one meal, or the like or a component of the one meal which is for animals to eat, ingest and digest, or is suitable therefor. The feed composition according to one embodiment may further comprise concentrated feed, and/or special feed. The concentrated feed may be seed fruits including grains such as wheat, oats, corn and the like, bran including rice bran, wheat bran, barley bran, and the like as a by-product obtained by refining grains, sesame dregs which are by-products obtained by drilling soybeans, fluids, sesame seeds, linseeds, coco palms, and the like for oil, and residues such as residual starches which are the main component of starch remnants, which are the remainder after removing starch from sweet potatoes and potatoes and the like, fish soluble, which is a concentrated product of fresh liquid obtained from fish meal, fish residues and fish, animal feed such as meat meal, blood meal, feather meal, skimmed milk powder, dried whey which is a residual liquid when cheese is prepared from milk, and casein is prepared from skimmed milk, and the like, yeast, chlorella, and/or seaweed, and the like.

The feed composition according to one embodiment may mean feed in a form that is finally consumed by an animal, a dietary supplement that can be mixed in feed, and/or a feed additive. The dietary supplementary is, for example, a composition containing a preparation which provides a therapeutic agent or a digestive agent to animals, and a composition that is not usually a source of calorie intake of a living body, that is, an energy source, but is ingested in addition to normal animal feed. The feed additive may refer to a substance added to feed for various effects such as nutrient supplement and weight loss prevention, improvement of digestibility of fiber in feed, oil quality improvement, reproduction disorder prevention and conception rate improvement, summer high temperature stress prevention, and the like. The feed to which the feed additive can be added may be selected from the group consisting of commercially available feed, or grains, root fruits, food processing by-products, algae, fibers, pharmaceutical by-products, oils and fats, starches, meal, grain by-products, proteins, inorganics, minerals, single cell proteins, zooplanktons, leftover food, and the like, but not limited thereto.

In one embodiment, the feed composition may be a feed additive, and when the feed additive according to one embodiment is mixed to feed (for example, mixed feed and/or ingredient feed finally ingested by animals), it may be added at a weight of 0.001% or more, 0.005% or more, 0.01% or more, 0.05% or more, 0.1% or more, 0.5% or more, 1% or less, 0.5% or less, 0.1% or less, 0.05% or less, 0.01% or less, 0.005% or less, 0.001 to 1%, 0.001 to 0.5%, 0.001 to 0.1%, 0.001 to 0.05%, 0.001 to 0.01%, 0.001 to 0.005%, 0.005 to 1%, 0.005 to 0.5%, 0.005 to 0.1%, 0.005 to 0.05%, 0.005 to 0.01%, 0.01 to 1%, 0.01 to 0.5%, 0.01 to 0.1%, 0.01 to 0.05%, 0.05 to 1%, 0.05 to 0.5%, 0.05 to 0.1%, 0.1 to 1%, 0.1 to 0.5%, or 0.5 to 1%, based on the total feed weight, and it may be mixed with a feed raw material, supplementary feed, a supplement, and/or other kinds of additives other than the active ingredient according to one embodiment, and the like.

By mixing and supplying the bacteriophage and anhydrous sodium sulfate (or a composition for improving stability of bacteriophage) to feed, bacteria in feed may be reduced or removed.

Other aspect, provides a method for manufacturing a feed composition, comprising mixing a bacteriophage and anhydrous sodium sulfate (or a composition for improving stability of bacteriophage). The feed composition is as described above.

Other aspect, provides a food composition comprising a bacteriophage and anhydrous sodium sulfate (or a composition for improving stability of bacteriophage). The bacteriophage specifically kills bacteria, so it may be used in treatment of processed food or non-processed food or food ingredients for prevention of bacterial infection. The food composition according to one embodiment may mean meat, sausage, bread, chocolate, candy, snacks, confectionery, pizza, ramen, other noodles, gum, dairy products including ice cream, various kinds of soups, beverages, tea, drinks, alcoholic beverages, vitamin complexes, health functional food and health food, and the like, and include all foods in a conventional sense.

By mixing and supplying the bacteriophage and anhydrous sodium sulfate (or a composition for improving stability of bacteriophage) to food, bacteria in food may be reduced or removed.

Other aspect, provides a method for manufacturing a food composition, comprising mixing a bacteriophage and anhydrous sodium sulfate (or a composition for improving stability of bacteriophage). The food composition is as described above.

Other aspect, provides a pharmaceutical composition for prevention or treatment of infection of bacteria of the genus of Escherichia, Salmonella, or Clostridium, or disease caused by bacteria of the genus of Escherichia, Salmonella, or Clostridium, comprising a bacteriophage and anhydrous sodium sulfate (or a composition for improving stability of bacteriophage). Other embodiment provides a method for prevention or treatment of infection of bacteria of the genus of Escherichia, Salmonella, or Clostridium, or disease caused by bacteria of the genus of Escherichia, Salmonella, or Clostridium, comprising administering a pharmaceutically effective dose of the bacteriophage and composition for improving stability of bacteriophage to a subject in need of prevention or treatment of infection of bacteria of the genus of Escherichia, Salmonella, or Clostridium, or disease caused by bacteria of the genus of Escherichia, Salmonella, or Clostridium. The bacteriophage specifically kills bacteria (for example, bacteria of the genus of Escherichia, Salmonella, or Clostridium), so it may be usefully used in prevention, or treatment of disease caused thereby.

The Escherichia sp. bacteria may be *E*. *coli, E. albertii, E. fergusonii, E. hermannii,* or *E*. *vulneris,* or the like, and the Salmonella sp. bacteria may be *S*. *bongori* or *S*. *enterica,* or the like, and the Clostridium sp. bacteria may be *C*. *botulinum, C. tetani, C. perfringens, C. hisolyticum, C. sordellii,* or *C*. *butyricum,* or the like, but not limited thereto.

The infection of the Escherichia sp. bacteria or disease caused thereby may be enteritis, Crohn's disease, ulcerative colitis, bacterial dysentery, urinary tract infection, skin infection, bacteremia, or sepsis, or the like, and the infection of the Salmonella sp. bacteria or disease caused thereby may be salmonellosis, Salmonella food poisoning, or the like, and the infection of the Clostridium sp. bacteria or disease caused thereby may be Botulinus food poisoning, Clostridium perfringens food poisoning, Clostridium difficile induced colitis, Clostridium necrotizing enterocolitis, neutropenic enterocolitis, or neonatal necrotizing enterocolitis, but not limited thereto.

The pharmaceutical composition according to one embodiment may further comprise an appropriate carrier, excipient or diluent commonly used for manufacture of a pharmaceutical composition. Specifically, the pharmaceutical composition may be used as formulated in a form of oral formulations such as powder, granules, tablets, capsules, suspension, emulsion, syrup, aerosols, and the like, external preparations, suppositories and sterile injection solution, according to common methods, respectively.

The pharmaceutical composition according to one embodiment may be administered in a pharmaceutically effective dose, and in the present application, "pharmaceutically effective dose" means an amount sufficient to treat or prevent disease with a reasonable benefit/risk ratio applicable to medical treatment or prevention, and the effective dose level may be determined according to factors including severity of disease, activity of a drug, patient's age, body weight, health, gender, sensitivity to a drug of a patient, administration time, an administration route and an excretion rate of the composition of the present application used, treatment period, a drug used in combination or simultaneous use with the composition of the present application used and other factors well known in the medical field. The pharmaceutical composition according to one embodiment may be administered as an individual therapeutic agent, or administered in combination with other therapeutic agent, and may be administered sequentially or simultaneously with a conventional therapeutic agent. In addition, it may be single or multiple administration. It is important to administer an amount capable of obtaining the maximum effect with the minimum amount without side effects in consideration of all the above factors.

The dose of the pharmaceutical composition according to one embodiment may be administered by 0.0001 to 10,000mg/g, 0.0001 to 5,000mg/g, 0.0001 to 1,000mg/g, 0.0001 to 500mg/g, 0.0001 to 100mg/g, 0.0001 to 50mg/g, or 0.0001 to 10mg/g per day to a mammal, but not limited thereto. The administration frequency of the pharmaceutical composition of the present application is not particularly limited thereto, but it may be administered once a day or administered several times by dividing the dose. The dose is not intended to limit the scope of the present application in any way,

The subject of administration of the pharmaceutical composition provided in the present description may be a mammal including humans, dogs, cats, horses, pigs, goats, rabbits, mice, rats, and the like, or a cell, tissue, or culture thereof isolated therefrom, and in one embodiment, the subject may be a subject (mammal such as a human, and the like) which requires prevention, improvement and/or treatment of disease as described above, or a cell, tissue or culture thereof isolated therefrom. The disease may be infection of E. coli, Salmonella, or Clostridium sp. bacteria, or a disease caused by E. coli, Salmonella, or Clostridium sp. bacteria.

Other aspect, provides a method for manufacturing a pharmaceutical composition, comprising mixing a bacteriophage and anhydrous sodium sulfate (or a composition for improving stability of bacteriophage).

Other aspect, provides an antibacterial composition comprising a bacteriophage and anhydrous sodium sulfate (or a composition for improving stability of bacteriophage).

In the present description, the antibacterial activity may mean activity of inhibiting and/or killing growth of bacteria, or the like, and the antibacterial composition may mean a composition having the antibacterial activity. The antibacterial activity may be used in combination with antibiotic activity, and the like, and the antibacterial composition encompasses all types of preparations having inhibiting ability and/or killing ability of growth of bacteria, and unless otherwise mentioned, it may be interchangeably used with an antibacterial agent, an antibiotic agent, a preservative, a bactericide, a disinfectant, and the like.

In one embodiment, the antibacterial composition collectively refers to a preparation for preventing bacterial infection, and may be used for a general life disinfectant, a disinfectant for food and cooking places and facilities, disinfection of various growth products such as buildings including chicken farms and barns, and the like, livestock, drinking water, litter, egg beds, transport vehicles, tableware and the like. The bacteria may be bacteria of the genus of E. coli, Salmonella, or Clostridium, but not limited thereto.

Other aspect, provides a method for manufacturing an antibacterial composition, comprising mixing a bacteriophage and anhydrous sodium sulfate (or a composition for improving stability of bacteriophage). The antibacterial composition is as described above.

Other aspect, provides a method for improving bacteria stability, comprising mixing a bacteriophage and anhydrous sodium sulfate (or a composition for improving stability of bacteriophage). The bacteriophage or improving stability of bacteriophage is as described above.

Other aspect, provides an antibacterial method, comprising a step of applying a bacteriophage and anhydrous sodium sulfate (or a composition for improving stability of bacteriophage) to a subject in need of antibacterial. The antibacterial method may be a control method of bacteria (a method for controlling bacteria), a reducing method of bacteria (a method for reducing bacteria), an inhibiting method of bacteria (for example, a method for inhibiting bacterial growth, etc.), or a killing method of bacteria, or the like, but not limited thereto.

Other aspect, provides a washing method, comprising a step of applying a bacteriophage and anhydrous sodium sulfate (or a composition for improving stability of bacteriophage) to a subject in need of washing.

Other aspect, provides a use of anhydrous sodium sulfate for use in improvement of stability of bacteriophage, or manufacture of a composition for improving stability of bacteriophage.

Other aspect, provides a use of a bacteriophage and anhydrous sodium sulfate for use in manufacture of a feed composition, food composition, pharmaceutical composition, or antibacterial composition.

Other aspect, provides a use of a bacteriophage and a composition for improving stability of bacteriophage for use in manufacture of a feed composition, food composition, pharmaceutical composition, or antibacterial composition.

### [ADVANTAGEOUS EFFECTS]

The present application can improve storage stability of bacteriophage by using a composition for improving stability of bacteriophage comprising anhydrous sodium sulfate.

### [BRIEF DESCRIPTION OF THE DRAWINGS]

FIG. 1 shows a graph by measuring the titer of the bacteriophage on Day 0, Day 7 and Day 14 after storing bacteriophage samples (4g of the 5 kinds-mixed bacteriophage and excipient comprising no anhydrous sodium sulfate) under conditions of 50±2 °C, 90±5% RH.
FIG. 2 shows a graph by measuring the titer of the bacteriophage on Day 0, Day 7 and Day 14 after storing bacteriophage samples (4g of the 5 kinds-mixed bacteriophage and excipient comprising anhydrous sodium sulfate 20%(w/w)) under conditions of 50±2 °C, 90±5% RH.
FIG. 3 shows a graph by measuring the titer of the bacteriophage on Day 0, Day 7 and Day 14 after storing bacteriophage samples (4g of the 5 kinds-mixed bacteriophage and excipient comprising anhydrous sodium sulfate 50%(w/w)) under conditions of 50±2 °C, 90±5% RH.
FIG. 4 shows a decrease in titer of the bacteriophage (Δlog(pfu/g)) measured on Day 0 and Day 14 after storing bacteriophage samples under conditions of 50±2 °C, 90±5% RH. As an excipient, an excipient comprising no anhydrous sodium sulfate, an excipient comprising anhydrous sodium sulfate 20%(w/w) and an excipient comprising anhydrous sodium sulfate 50%(w/w) were used.

### [MODE FOR INVENTION]

Hereinafter, the present invention will be described more specifically by the following examples. However, they are intended to illustrate the present invention only, but the scope of the present invention is not limited by these examples.

### Example 1. Evaluation of storage stability of anhydrous sodium sulfate

### 1. Bacteriophage sample manufacture

By mixing 4g of 5 kinds-mixed bacteriophage with 36g of an excipient, bacteriophage sample 1 to sample 3 were prepared by 2 bags per sample as Table 1 below. Sample 1 used an excipient prepared by mixing 90%(w/w) of wheat flour (Sajo Dongaone, wheat flour (2^{nd} grade, medium 2 class), 5%(w/w) of silica (FuJian SanMing Zheng Yuan Chemical, Precipitated Sillica, ZJ-516B), and 5%(w/w) of calcium carbonate (Omya Korea, calcium carbonate). Sample 2 and Sample 3 used a mixed excipient prepared by mixing the excipient component prepared by mixing wheat flour, silica and calcium carbonate as above (conventional excipient) and anhydrous sodium sulfate. The composition of Sample 1 to Sample 3 was summarized in Table 1 below.

The 5 kinds-mixed bacteriophage was prepared with the following composition:
CJ7: 6×10⁹ PFU/g, CJ11: 4×10⁹ PFU/g, CJ23: 1×10⁹ PFU/g, CJ24: 5×10⁹ PFU/g, CJ22: 3×10⁸ PFU/g.

**[Table 1]**

| Bacteriophage sample | Composition |
|---|---|
| Sample 1 | 4 g of 5 kinds-mixed bacteriophage 36 g of excipient (conventional excipient 100%(w/w)) |
| Sample 2 | 4 g of 5 kinds-mixed bacteriophage 36 g of excipient (conventional excipient 80%(w/w)+anhydrous sodium sulfate 20%(w/w)) |
| Sample 3 | 4 g of 5 kinds-mixed bacteriophage 36 g of excipient (conventional excipient 50%(w/w)+anhydrous sodium sulfate 50%(w/w)) |

The trivial name, target strain and source of acquisition of the bacteriophage are as Table 2 below:

**[Table 2]**

| Experim ental group | Phage trivial name | Target strain | Accession number |
|---|---|---|---|
| CJ7 | Siphoviridae | *Salmonella spp.* | KCCM11030P |
| CJ11 | Siphoviridae | *Salmonella spp.* | KCCM11208P |
| CJ23 | Podoviridae | *Escherichia coli* | KCCM11365P |
| CJ24 | Siphoviridae | *Escherichia coli* | KCCM11462P |
| CJ22 | Siphoviridae | *Clostridium perfringens* | KCCM11364P |

### 2. Storage condition

40 g of each sample was placed in an aluminum bag and heat sealing was performed 3 times. Each sample was stored under a condition of 50±2 °C, 90±5% RH (relative humidity).

### 3. Phage titer measurement

In 7 days and 14 days after sample storage, each sample was diluted by 10 times in SM buffer (5.8 g/L NaCl, 2 g/L MgSO₄.7H₂O, 0.05 M Tris-Cl (pH 7.5)) (sample 20 g + SM buffer 180 ml). After centrifugation (10,000 rpm, 10 minutes) of 50 ml of the diluted solution, the bacteriophage solution was filtered with a 0.2 µm filter.

### 4. Plaque assay titration

After serial dilution of the bacteriophage solution to an appropriate concentration, 100 µl of the bacteriophage sample diluted in 5 ml of 0.7% soft agar (BD DIFCO, Cat. No. 44164) and 50 µl of an indicator strain by phage (OD_{600 nm}=2) were mixed.

After pouring in a general LB (Luria-Bertani, aerobic) or BHI (Brain Heart Infusion, anaerobic) medium and plating, the number of plaques after culturing in an incubator (aerobic; 30 °C, 18h, anaerobic; 37 °C, 18h) was measured to quantify the bacteriophage.

### 5. Experimental result

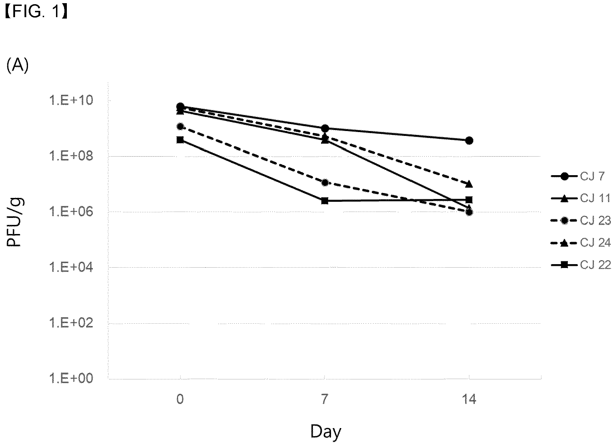

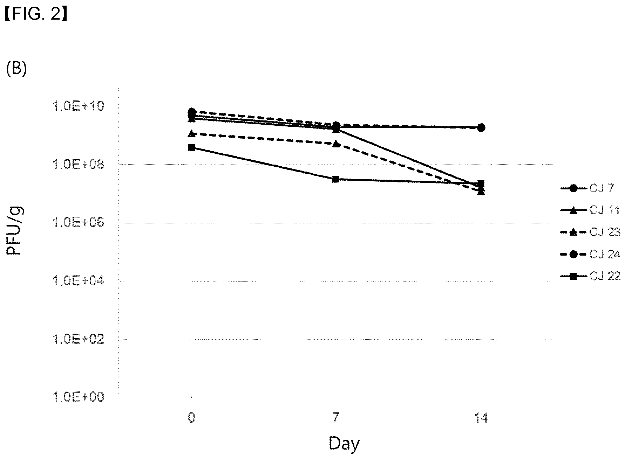

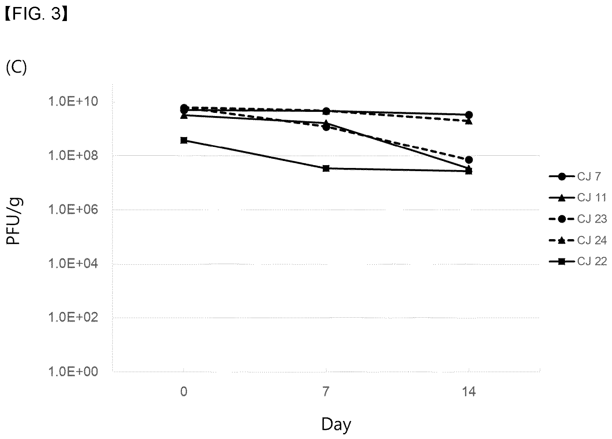

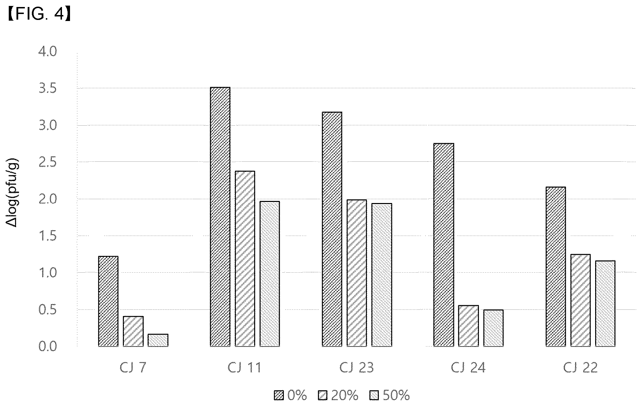

In 7 days and 14 days after sample storage, the result of quantifying the bacteriophage of each sample was shown in FIGs. 1 to 3 and Table 2 to Table 4 (Sample 1: FIG. 1, Table 2; Sample 2: FIG. 2, Table 3; Sample 3: FIG. 3, Table 4). The difference between the titer value (log(pfu/g)) on Day 0 and the titer value (log(pfu/g)) on Day 14 was expressed as Δlog, and this was divided by the titer value on Day 0 to describe a titer decrement. In FIG. 4, the titer decrement (Δlog) in 14 days after sample storage of the 5 kinds of bacteriophage (CJ7, CJ11, CJ23, CJ24, CJ22) comprised in Sample 1 to Sample 3 was shown as a graph.

**[Table 3]**

| Sample1 | 0 day (pfu/g) | 14 day (pfu/g) | 0 day log(pfu/g) | 14 day log(pfu/g) | Δlog | Titer decrement |
|---|---|---|---|---|---|---|
| CJ 7 | 6.1E+09 | 3.7E+08 | 9.8 | 8.6 | 1.2 | 12.24% |
| CJ 11 | 4.3E+09 | 1.3E+06 | 9.6 | 6.1 | 3.5 | 36.46% |
| CJ 23 | 1.2E+09 | 1.0E+06 | 9.1 | 6.0 | 3.1 | 34.07% |
| CJ 24 | 5.6E+09 | 1.0E+07 | 9.7 | 7.0 | 2.7 | 27.84% |
| CJ 22 | 3.8E+08 | 2.7E+06 | 8.6 | 6.4 | 2.2 | 25.58% |

**[Table 4]**

| Sample 2 | 0 day (pfu/g) | 14 day (pfu/g) | 0 day log(pfu/g) | 14 day log(pfu/g) | Δlog | Titer decrement |
|---|---|---|---|---|---|---|
| CJ 7 | 4.9E+09 | 1.9E+09 | 9.7 | 9.3 | 0.4 | 4.12% |
| CJ 11 | 3.9E+09 | 1.7E+07 | 9.6 | 7.2 | 2.4 | 25.00% |
| CJ 23 | 1.2E+09 | 1.2E+07 | 9.1 | 7.1 | 2.0 | 21.98% |
| CJ 24 | 6.7E+09 | 1.9E+09 | 9.8 | 9.3 | 0.6 | 6.12% |
| CJ 22 | 3.9E+08 | 2.2E+07 | 8.6 | 7.3 | 1.2 | 13.95% |

**[Table 5]**

| Sample 3 | 0 day (pfu/g) | 14 day (pfu/g) | 0 day log(pfu/g) | 14 day log(pfu/g) | Δlog | Titer decrement |
|---|---|---|---|---|---|---|
| CJ 7 | 4.9E+09 | 3.4E+09 | 9.7 | 9.5 | 0.2 | 2.06% |
| CJ 11 | 3.2E+09 | 3.4E+07 | 9.5 | 7.5 | 2.0 | 21.05% |
| CJ 23 | 6.1E+09 | 7.2E+07 | 9.8 | 7.9 | 1.9 | 19.39% |
| CJ 24 | 6.1E+09 | 2.0E+09 | 9.8 | 9.3 | 0.5 | 5.10% |
| CJ 22 | 3.8E+08 | 2.6E+07 | 8.6 | 7.4 | 1.2 | 13.95% |

As a result of quantification, when using the conventional excipient (Sample 1), the 5 kinds of bacteriophage (CJ7, CJ11, CJ23, CJ24, CJ22) in the excipient showed a bacteriophage titer decrease (average decrease of 27.2%) with an average 2.5 log, when exposed at 50±2 °C, 90±5% RH for 14 days (FIG. 1, Table 3).

On the other hand, when the mixed excipient in which an excipient and anhydrous sodium sulfate were mixed was used, the 5 kinds of bacteriophage in the excipient showed a bacteriophage titer decrease with about 1.3 log (average decrease of 14.2%) (Sample 2, FIG. 2, Table 4), and 1.2 log (average decrease of 12.3%) (Sample 3, FIG. 3, Table 5), respectively.

In other words, compared to the conventional excipient, when using the mixed excipient in which anhydrous sodium sulfate was mixed, an effect of maintaining stability of bacteriophage of average 1.3 log was shown.

From the above description, those skilled in the art to which the present invention pertains will understand that the present invention may be embodied in other specific forms without changing its technical spirit or essential features. In this regard, the examples described above should be understood as illustrative not restrictive in all respects. The scope of the present invention should be construed as including all changed or modified forms derived from the meaning and scope of claims described below and equivalents thereof rather than the detailed description above.

## Claims

1. A composition for improving stability of bacteriophage comprising anhydrous sodium sulfate.

2. The composition for improving stability of bacteriophage according to claim 1, comprising 5 to 50% by weight of anhydrous sodium sulfate.

3. The composition for improving stability of bacteriophage according to claim 1, which is for improving stability of at least one bacteriophage selected from the group consisting of Myoviridae, Siphoviridae, and Podoviridae bacteriophage.

4. The composition for improving stability of bacteriophage according to any one of claims 1 to 3, for use in manufacture of a feed, food or antibacterial composition.

5. A feed composition, comprising a bacteriophage and the composition for improving stability of bacteriophage according to any one of claims 1 to 3.

6. The feed composition according to claim 5, wherein the mixing ratio of the bacteriophage and composition for improving stability of bacteriophage comprised in the feed composition is 1: 9 to 1: 99 (above, weight of bacteriophage: weight of composition for improving stability of bacteriophage).

7. The feed composition according to claim 5, wherein the feed composition has,
a titer of the bacteriophage comprised in the feed composition that is 10% or more higher than a feed composition not comprising anhydrous sodium sulfate,
when stored under conditions of a temperature of 45 to 60 °C and a relative humidity of 80 to 100% for 1 day to 14 days.

8. A food composition, comprising a bacteriophage and the composition for improving stability of bacteriophage according to any one of claims 1 to 3.

9. The food composition according to claim 8, wherein the mixing ratio of the bacteriophage and composition for improving stability of bacteriophage comprised in the food composition is 1: 9 to 1: 99 (above, weight of bacteriophage: weight of composition for improving stability of bacteriophage).

10. The food composition according to claim 8, wherein the food composition has,
a titer of the bacteriophage comprised in the food composition that is 10% or more higher than a food composition comprising no anhydrous sodium sulfate,
when stored under conditions of a temperature of 45 to 60 °C and a relative humidity of 80 to 100% for 1 day to 14 days.

11. An antibacterial composition, comprising a bacteriophage and the composition for improving stability of bacteriophage according to any one of claims 1 to 3.

12. The antibacterial composition according to claim 11, wherein the mixing ratio of the bacteriophage and composition for improving stability of bacteriophage comprised in the antibacterial composition is 1: 9 to 1: 99 (above, weight of bacteriophage: weight of composition for improving stability of bacteriophage).

13. The antibacterial composition according to claim 11, wherein the antibacterial composition has,
a titer of the bacteriophage comprised in the antibacterial composition that is 10% or more higher than an antibacterial composition comprising no anhydrous sodium sulfate,
when stored under conditions of a temperature of 45 to 60 °C and a relative humidity of 80 to 100% for 1 day to 14 days.

14. A method for improving stability of bacteriophage, comprising a step of mixing a bacteriophage and the composition for improving stability of bacteriophage according to any one of claims 1 to 3.

15. An antibacterial method, comprising a step of applying a bacteriophage and the composition for improving stability of bacteriophage according to any one of claims 1 to 3, to a subject in need of antibacterial.
